# EUROPEAN PATENT APPLICATION

(11) **EP 0 649 899 A1**
(43) Date of publication of application: **26.04.1995**
(21) Application number: 94307780.0
(22) Date of filing: 24.10.1994
(51) Int. Cl.: C12N 1/14, C12N 1/38

(54) **Novel method for culturing filamentous fungi, carrier for immobilizing filamentous fungi and immobilized fungus composition for culturing**

(30) Priority: 25.10.1993 JP 288753/93
(71) Applicant: MIZUSAWA INDUSTRIAL CHEMICALS, LTD., Chuo-ku Tokyo (JP)
(72) Inventor: Kiyoshi, Abe, c/o Mizusawa Ind. Chem. Ltd., Tokyo (JP); Yasuo, Mizoguchi, c/o Mizusawa Ind. Chem. Ltd., Tokyo (JP)
(74) Representative: Cresswell, Thomas Anthony

(57) **Abstract**

A process of culturing a filamentous fungus which comprises adding a chain clay mineral carrier to a culture medium containing the fungus or bringing the fungus immobilized on a chain clay mineral carrier into contact with a culture medium.

## Description

### BACKGROUND OF THE INVENTION

### (Field of the Invention)

The present invention relates to a method of producing so-called useful substances or of treating waste liquids based upon culturing and fermentation by using filamentous fungi such as mold, actinomyces and the like bacteria, as well as to a carrier for immobilizing filamentous fungi that is used for the above method and an immobilized fungus composition for culturing.

In particular, the present invention is to improve the production of useful substances by controlling the size of mass of filamentous fungi, by increasing the rate of migration of substances such as oxygen and substrates and by improving the separation of fungi based upon the addition of a chain clay mineral carrier such as sepiolite of a formanite-type clay mineral to the culture solution. The present invention further attempts to apply the method to a process for the production by fermentation of useful substances from oils and fats as starting materials and for efficiently treating waste materials and waste waters containing animal and plant oils such as edible oils by effectively utilizing the oil-adsorbing property of a carrier composed of the formanite-type clay.

### (Description of the Prior Art)

The so-called filamentous fungi such as mold and actinomyces produce a wide range of useful substances like organic acids such as citric acid, gluconic acid and itaconic acid, antibiotics such as penicillin, cephalosporin C, chlorotetracycline and cephamycin C, as well as enzymes such as β-galactosidase, gluco amylase and lipase.

However, the culture solution of these filamentous fungi has a high viscosity since fungi are entangling with each other, and the rate of feeding oxygen and substrates is limited causing the rate of production by fermentation and yields to be decreased. In recovering and refining the products formed by fermentation, furthermore, the fungi must be isolated from the culture solution by centrifuge or filtration. However, the culture solution of filamentous fungi has a viscosity which is so high that the isolation of fungi is very difficult imposing a serious hindrance upon the production on a commercial scale.

In recent years, a process using immobilized microorganisms has been reported as a biocatalyst for the bioreactor. As a carrier for immobilization, there has been used an organic carrier such as calcium alginate or cellulose polyamide acrylate. These carrier materials, however, involve many problems from the standpoint of physical strength and chemical and biological resistance, and have not almost been put into practical use regardless of their results of study.

Use of inorganic carriers such as diatomaceous earth and the like has been reported as an agent for immobilizing microorganisms. It is not, however, obvious from their structure whether they are working as a carrier for immobilizing filamentous fungi (they may be in a state of simply existing together).

### SUMMARY OF THE INVENTION

The principal object of the present invention is to improve properties of the culture solution by adding an effective carrier in the fermentation of filamentous fungi where the viscosity of the culture solution becomes inevitably high, to improve migration of substances such as oxygen and substrates, to increase the rate of production by fermentation, to increase the yields, as well as to improve separation of fungi in the recovery and refining of the products produced by fermentation yet minimizing the cost required for the recovery and refining.

In order to achieve the above-mentioned object, the present invention provides a method of culturing filamentous fungi such as mold and actinomyces by adding a carrier of a chain clay mineral to a culture medium containing filamentous fungi or by bringing filamentous fungi immobilized on the carrier of a chain clay mineral into contact with a culture medium.

The filamentous fungi can be effectively cultured by bringing the filamentous fungi into contact with the culture medium by any one of a batch method, a half-batch method, a repeated batch method or a continuous method.

The present invention makes it possible to produce useful substances in the culture medium to efficiently obtain useful substances by separating the filamentous fungi immobilized on the carrier of a chain clay mineral from the culture medium that contains the useful substances, as well as to efficiently decompose oils and fats by using waste materials or waste waters containing oils and fats and separating the filamentous fungi immobilized on the carrier of a chain clay mineral from the culture medium containing the decomposed products.

According to the present invention, it is desired that the carrier of a chain clay mineral is composed of a formanite-type clay mineral. Most desirably, sepiolite should be selected out of the clay minerals since it is produced and is available everywhere in the world. Though there is no particular limitation on the form of the clay, it is desired that the clay is of a fibrous form as observed by naked eyes or is of a form of converged fibers as observed through a microscope.

According to another embodiment of the present invention, there is provided a carrier for immobilizing filamentous fungi composed of formanite-type clay minerals, wherein the formanite-type clay is of a fibrous form or of a form of converged fibers, and has a fiber diameter of from 70 to 400 angstroms and a fiber length of from 0.2 to 400 µm.

According to a further embodiment of the present invention, there is provided an immobilized fungus composition for culturing comprising a carrier of a formanite-type clay mineral having a fiber diameter of from 70 to 400 angstroms and a fiber length of from 0.2 to 400 µm, and filamentous fungi of an amount of from 0.1 to 30% by weight immobilized onto said carrier.

According to the present invention, it was discovered that the filamentous fungi can be very effectively cultured in an environment where they are immobilized by adding a carrier of a chain clay mineral such as a sepiolite-type carrier to the culture solution. Furthermore, addition of this carrier helps greatly improve the separation of fungi.

That is, according to the present invention, the capacity of the apparatus is minimized and a filtering assistant is used in a minimum amount in a step of separating the fungi in a process of recovering and refining the fermentation product owing to the improved separation of fungi, making it possible to greatly decrease the cost required for the recovery and refining. Moreover, the improved separation of fungi contributes greatly to enhancing the probability of realizing the repeated bach culturing.

In general, the production by fermentation is in many cases carried out by bach culturing. After the fermentation is completed, the fungi are separted and are disposed of. That is, the fungi are not used again, and the substrates used for the synthesis of fungi are wasted each time requiring a considerable cost for the disposal of the fungi. Accordingly, it is desired to reuse the fungi as much as possible.

The repeated batch fermentation was developed to meet such needs. According to this method, the fungi are separted by some means after the fermentation has been completed, and a fresh culture medium is added thereto to effect the fermentation again. The fungi are separted by centrifuge or press filtering. The filamentous fungi by their own nature can be separted very poorly, and the apparatus for separtion inevitably becomes large in size requiring increased cost for facilities. Besides, the probability increases in which the filamentous fungi are contaminated.

As described above, the repeated batch culture is expected for its effects in theory but is not suited for being put into practical use on an industrial scale. Addition of the carrier of a chain clay mineral such as the sepiolite-type carrier is effective in solving the above-mentioned problem, enabling the carrier to be stably used for extended periods of time and making it possible to realize the repeated batch culture because of its excellent physical strength and excellent chemical and biological durability.

Moreover, the properties in that the chain clay mineral carrier such as the sepiolite-type carrier selectively adsorbs oils have very diversified the process for utilizing microorganisms. That is, some fermentation products use oils and fats as a sole carbon source. Moreover, oil-and-fat refining industries and food industries are encountering the process for treating large amounts of waste materials and waste waters that contain oils and fats. When the above-mentioned carrier is added to a microorganism reaction process that uses oils and fats as a sole carbon source, microorganisms, oils and fats are simultaneously adsorbed, and the fermentation is promoted. Similar effects can be expected even in the treatment with microorganisms of waste materials or waste waters containing oils and fats.

### BRIEF DESCRIPTION OF THE DRAWINGS

Fig. 1 is a graph showing the results of production of itaconic acid by batch fermentation according to Example 1;
Fig. 2 is a graph showing the results of production of itaconic acid by repeated batch fermentation using Aidplus A® according to Example 2;
Fig. 3 is a graph showing the results of production of itaconic acid by repeated batch fermentation using Aidplus B® according to Example 2;
Fig. 4 is a graph showing the results of production of itaconic acid by batch fermentation using a cellulose-type carrier according to Example 2;
Fig. 5 is a graph showing the results of production of cephamycin C by batch fermentation when cultured in a flask;
Fig. 6 is a graph showing the results of production of a chlorotetracycline by batch fermentation;
Fig. 7 is a graph showing the results of production of gluco amylase by repeated batch fermentation using a packed bed reactor;

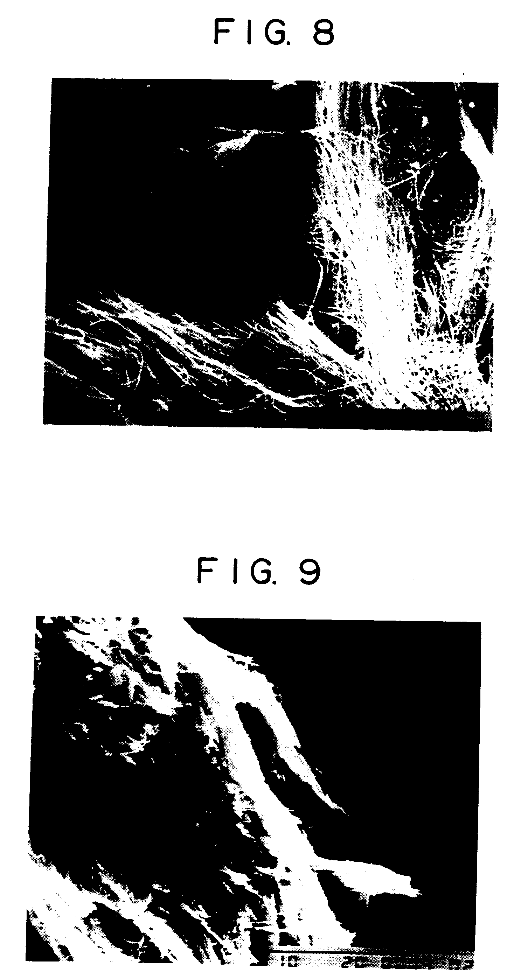

Fig. 8 is an electron microphotograph showing, at a magnification of 2000 times, the fibrous structure of a sepiolite-type carrier used in the present invention and the structure of converged fibers subjected to the opening;
Fig. 9 is an electron microphotograph showing, at a magnification of 2000 times, a state in which the fibers of the sepiolite-type carrier used in the present invention are strongly converged;

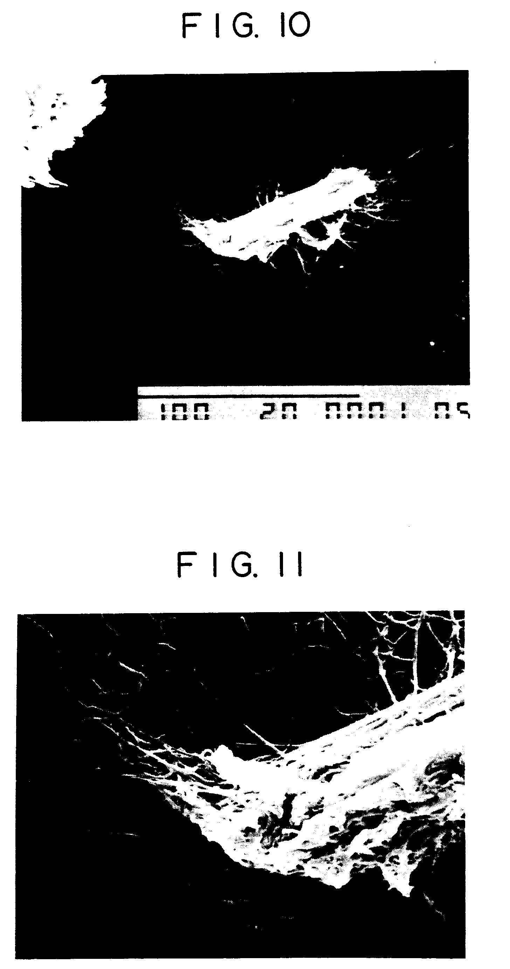

Fig. 10 is an electron microphotograph showing, at a magnification of 1000 times, the fibrous structure of fungi immobilized on the sepiolite-type carrier;

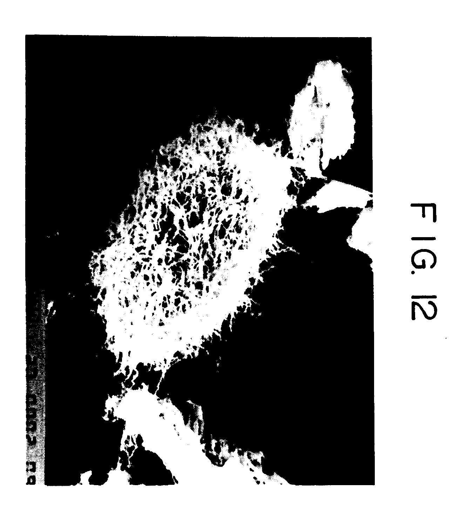

Fig. 11 is an electron microphotograph showing, at a magnification of 2000 times, the fibrous structure of fungi immobilized on the sepiolite-type carrier; and
Fig. 12 is an electron microphotograph showing, at a magnification of 1000 times, the state where filamentous fungi are liberated alone like filaments.

### DETAILED DESCRIPTION OF THE INVENTION

The chain clay minerals used in the present invention are magnesium silicate clay minerals which are of a fibrous form or a converged form in appearance as represented by sepiolite, attapulgite and palygorskite which are formanite clays. They are porous chain clay minerals having a three-dimensional chain structure and having such a large specific surface area that the holes formed among the gaps of the chain structure lie over a range of from 100 to 350 m²/g as measured by the BET method unlike the two-dimensional layered crystalline structure such as talc, and further exhibit adsorptive action.

Furthermore, sepiolite and the like have a distinguished feature in that they do not swell in an aqueous system, unlike the ordinary layered clay minerals as represented by montmorillonite that is a porous clay mineral.

Owing to the above-mentioned features, i.e., owing to the fibrous form and porous nature, the chain clay minerals such as sepiolite and the like permit the filamentous fungi to be favorably entangled compared with other ordinary clay minerals and other inorganic carriers, enable the filamentous fungi to be strongly and stably immobilized, render the immobilized fungi to become porous, help feed nutrient substrates and oxygen to the fungi, and help improve filtering property.

According to the present invention, it is desired that the chain clay minerals are subjected to a primary weak pulverization such as kneading and pulverization by a pulverizer or a super mixer, milling using a ball mill or a hammer mixer, or impact pulverization using a jet mill in order to partly open the chain structure of the chain clay minerals of the converged form or to enlarge the gap of the chain structure prior to using the chain clay minerals as a carrier.

According to the present invention, the chain clay minerals can be used alone or can be used in combination with fibrous minerals of a two-layer structure such as halloysite or asbestos or in combination with volcanic fibrous minerals that include Kanuma earth, red earth or the like earth. As required, furthermore, the fibrous clay minerals may be used in combination with adsorptive clay minerals as represented by zeolite or acidic white earth or in combination with such rocks as cristobarite, quartz, feldspar and the like.

According to the present invention, furthermore, the chain clay minerals may, as required, be molded alone or together with other inorganic compounds and organic compounds into columns, rings, pellets or sheets after having been subjected to the above-mentioned primary pulverization in order to use them as the carrier.

The sepiolite that is preferably used in the present invention has a chemical structure expressed by the formula,

(OH₂)₄(OH)₄Mg₈Si₁₂O₃₀ · 6-8H₂O

and has a chain crystal structure in which bricks are alternately stacked to fabricate a two-dimensional crystal structure like talc.

Due to the holes formed among the gaps of the chain, furthermore, the sepiolite has a large specific surface area and adsorptive property despite of its fibrous form, which are distinguished features that are not obtained with other fibrous minerals.

The present invention desirably uses the sepiolite having a specific surface area over a range of from 100 to 350 m²/g and an oil absorption amount over a range of from 100 to 300 ml/100 g. When the specific surface area is smaller than this range, the porosity is not sufficient and when the specific surface area is larger than this value, no further improved effect is obtained.

It is desired that the sepiolite of the fibrous form or the converged form desirably used in the present invention, usually, has a fiber diameter of from 70 to 400 angstroms and a fiber length of from 0.2 to 400 angstroms.

When the fiber diameter and the fiber length are smaller than the above ranges, it becomes difficult to obtain a fungus composition for culturing that exhibits the aforementioned excellent properties.

The following Table 1 shows a general chemical composition of the sepiolite (dried at 110 °C for 2 hours) that is placed in the market.

**Table 1**

| General chemical composition of sepiolite | |
|---|---|
| | (% by weight) |
| SiO₂ | 52.50 |
| MgO | 22.8 |
| Al₂O | 1.7 |
| Fe₂ O₃ | 0.8 |
| CaO | 0.8 |
| H₂ O⁺ | 10.5 |
| H₂ O⁻ | 11.0 |

There is no particular limitation on the microorganisms used in the present invention provided they are filamentous fungi. There can be used, for instance, Aspergillus terreus that produces itaconic acid, Rizopus oryzae that produces lactic acid, Aspergillus niger that produces citric acid, gluconic acid and β -galactosidase, Aspergillus awamori that produces gluco amylase, Streptomyces clavuligenes that produces cephamycin C which is an antibiotic, Streptomycesaureofaciens that produces chlorotetracycline, Cephalosporuium polyaleuruim that produces cephalosporin C, and the like microorganisms.

Concretely speaking, there can be used Aspergillus terreus NRRL-1960 that produces itaconic acid, Aspergillus niger NRRL-567 that produces citric acid, Aspergillus niger IAM-2094 that produces gluconic acid, Aspergillus awamori IFO-5708 that produces gluco amylase, Aspergillus niger NRRL-31125 that produces β-galactosidase, Stereptomyces clavuligenes NRRL-3585 that produces cephamycin C, Streptomyces aureofaciens NRRL-2209 that produces chlorotetracycline, and Cephalosporuium polyaleurium ATCC-20359 that produces cephalosporin C.

The liquid culture medium used in the present invention may be any one in which filamentous fungi can be grown but to which the chain clay mineral carrier such as the sepiolite carrier is added.

The culture can be conducted by using a reactor of any type such as an ordinary ventilated agitation layer vessel, air lift bubble tower or packed bed reactor. Moreover, the culture can be conducted by any system such as bach system, half-batch system, repeated bach system or continuous system depending upon the strain that is used and properties of the fermented product.

According to the present invention as described above in detail, useful substances are efficiently produced by adding the chain clay mineral carrier such as the sepiolite carrier to the culture medium or by filling the reactor with fungi that are immobilized on the chain clay mineral carrier such as the sepiolite carrier. Enhanced fungi separation performance helps decrease the cost of recovery and refining. By utilizing the oil-adsorbing property of the carrier, furthermore, the invention tries to transform microorganisms that use oils and fats as a sole carbon source into useful substances and to treat waste materials and waste waters that contain oils and fats.

### EXAMPLES

The present invention will now be described by way of Examples which are only illustrative of the invention and do not impose any limitation upon the invention.

### Example 1.

### (Production of itaconic acid by batch fermentation)

A loopful of slant of a strain sorted by single spore separation from Aspergillus terreus NRRL-1960 having ability of forming itaconic acid was planted in a 500-ml Erlenmeyer flask which contained 100 ml of a preculture medium (5.5% of glucose, 0.5% of ammon nitrate, 0.2% of magnesium sulfate and 0.3% of CSL were adjusted to pH 3.0 with 2N nitric acid and were sterilized under 1 kg/cm²G for 15 minutes), and was shake-cultured in a rotary shaker (170 rpm) at 30 °C for two days.

The preculture solution was added in an amount of 10% (v/v) to a main culture medium (5.5% of glucose, 0.5% of NH₄ NO₃, 0.18% of MgSO₄7H₂O and 0.18% of CSL were adjusted to pH 2 to 2.2 with 2N nitric acid, blended with 1 g of Aidplus A® and 1 g of AidplusB® as carriers which are trade names of sepiolite clay minerals that have been subjected to the primary milling by jet mill produced by Mizusawa Industrial Chemicals, Ltd. and were sterilized under 1 kg/cm²G for 15 minutes), and was shake-cultured in a rotary shaker (170 rpm) at 30 °C for seven days.

As a control, the strain was also cultured in the same manner as described above but without adding the carrier.

Here, Aidplus A® is of a white cotton-like form and has 30 to 45% by weight of SiO₂, 10 to 20% by weight of MgO, 15 to 25% by weight of CaO, 18 to 25% by weight of Ig loss, and 20 to 60 µm of fiber length. Aidplus B® is a pale yellowish powder and has 57 to 63% by weight of SiO₂, 20 to 25% by weight of MgO, 0.5 to 2% by weight of CaO, 8 to 12% by weight of Ig loss, and 5 to 10 µ m of fiber length.

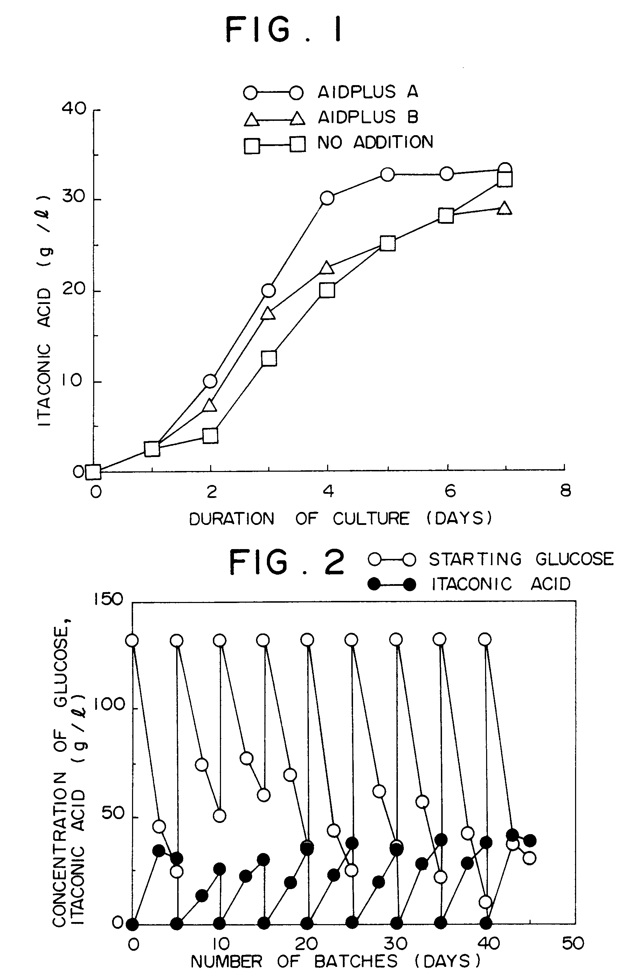

Fig. 1 shows the process of culture. Compared to no addition (control), Aidplus B® helps greatly increase the rate of producing itaconic acid. Itaconic acid was finally accumulated in an amount of 32.5 g/l. The yield was 60% with respect to the starting glucose.

Aidplus A® exhibited a large rate of production during the initial stage of culture as compared with the control. After the culture of seven days, however, the accumulated amount of itaconic acid was slightly smaller when Aidplus A® was used than that of the control.

Though the reason is not yet obvious why Aidplus B® promotes the production of itaconic acid, it is estimated that the addition of this carrier so changes the properties of the fermentation solution that the rate of mobility of oxygen is heightened.

### Example 2

### (Repeated batch culture of itaconic acid)

A loopful of the same slant as that of Example 1 was planted in a 500-ml Erlenmeyer flask that contained 100 ml of the preculture medium of fungi for producing itaconic acid prepared in the same manner as in Example 1 and Aidplus A® and Aidplus B® which are the carriers used in Example 1, and was shake-cultured in a rotary shaker (170 rpm) at 30 °C for two days.

The culture medium was subjected to the centrifuge by using a centrifugal separator (Model SCR 208, produced by Hitachi, Ltd.) at 400 rpm for 10 minutes to collect the fungi which were then suspended in the newly prepared main culture medium (containing the same components as the above-mentioned culture medium but increasing the amount of glucose to 13%) to carry out the culture in the same manner as described above. This operation was repeated to carry out batch culture.

As a control, batch culture was repeated in the same manner as described above but using a commercially available cellulose-type carrier (Fiber-Arm MIF-3, produced by Tokyo Rika Kikai Co.).

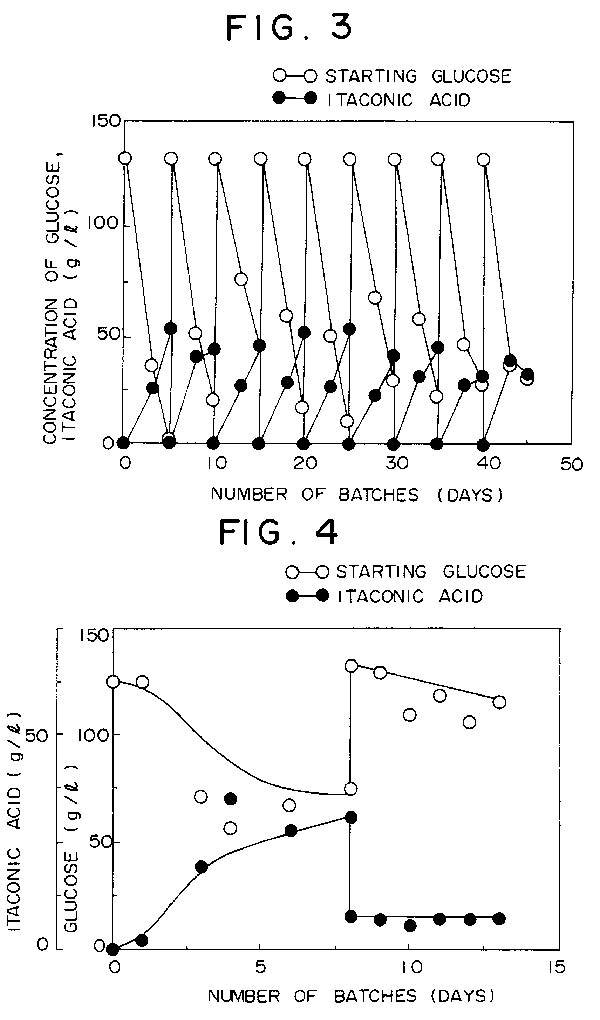

Fig. 2 illustrates the results of production by repeated batch fermentation by immobilizing itaconic acid-producing fungi onto Aidplus A®, Fig. 3 illustrates the results of production by repeated batch fermentation by immobilizing itaconic acid-producing fungi onto Aidplus B®, and Fig. 4 illustrates the results of production by repeated batch fermentation by immobilizing itaconic acid-producing fungi onto the cellulose-type carrier.

When the fungi are immobilized onto Aidplus A® as will be obvious from Fig. 2, glucose was consumed in an amount of 100 g/l in average and itaconic acid was produced in an amount of 35 g/l in average. The activity for producing itaconic acid lasted 45 days.

In the repeated batch culture in which the fungi were immobilized onto Aidplus B® as shown in Fig. 3, glucose was consumed in an amount of 105 g/l in average and itaconic acid was produced in an amount of as great as 50 g/l which was larger than the case of Aidplus A®. The activity for producing itaconic acid lasted 45 days.

In the case of the cellulose-type carrier used as a control, glucose was consumed in an amount of as small as 64 g/l even after the culture of 7 days, and itaconic acid was produced in an amount of 27.5 g/l. The immobilized fungi were separated by centrifuge and were suspended again in a culture medium to carry out the culture again. However, glucose was not consumed and itaconic acid was not almost produced, either. It was therefore evaluated that the cellulose-type carrier was not suited for the repeated batch culture.

### Example 3

### (Batch fermentation testing of antibiotic cephamycin C)

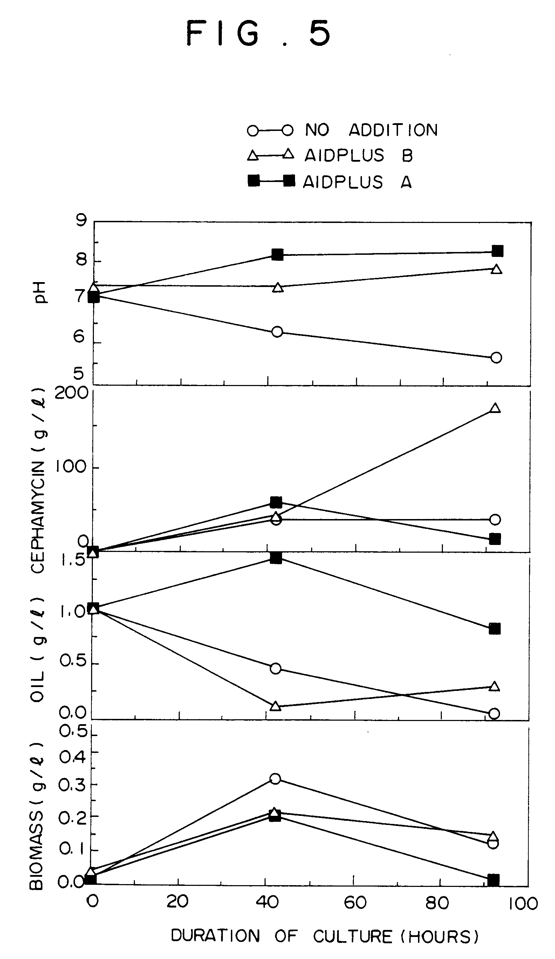

Aidplus A® and Aidplus B® that affect the fermentation of cephamycin C were compared with each other.
- Strain:: Streptomyces clavuligenes NRRL-3585
- Culture medium:: (Preculture) 4% of corn starch, 3% of soybean powder (Ajinomoto meat), 0.5% of CSL, 0.1% of magnesium sulfate, 0.1% of potassium diphosphate, 0.1% of ammonium sulfate, 0.02% of ferrous sulfate and 0.1% of common salt were adjusted to pH 7.0, and were sterilized in an autoclave under 1 kg/cm²G for 15 minutes, (main culture) 1.0% of soybean oil, 0.5% of polypeptone, 0.05% of magnesium sulfate, 0.1% of ammon nitrate, 0.05% of potassium phosphate, 1 g/l of the pretreated carrier were adjusted to pH 7.0 and were sterilized in an autoclave under 1 kg/cm²G for 20 minutes.
- Pretreatment of carrier:: Aidplus A® and Aidplus B® were washed three times with an N/5 acetic acid buffer solution, washed two times with distilled water, and were dried.
- Culturing method:: A loopful of the slant of the above-mentioned strain was inoculated to a 500-ml Erlenmeyer flask and was shake-cultured in a rotary shaker (170 rpm) at 30 °C for two days to use it as a seed fungi solution. The above seed fungi solution was planted in an amount of 5% (v/v) into 500-ml Erlenmeyer flasks containing 100 ml of the main culture medium containing each of the carriers, and was shake-cultured in a rotary shaker (170 rpm) at 30 °C for three days. As a control, the culture was carried out even in a culture medium without containing the carrier.
- Results:: Fig. 5 shows the process of culture. When Aidplus B® was used, cephamycin C was produced in an amount very larger than that of the control. Aidplus A® that is the same sepiolite-type carrier rather suppressed the production of the cephamycin C. Though the cause is not obvious, it is estimated that the pH value of the culture solution is considerably high in the case of Aidplus A® affecting the production of the fungi and, eventually, decreasing the yield of the cephamycin C.

### Example 4

### Production of antibiotic chlorotetracycline by batch fermentation.

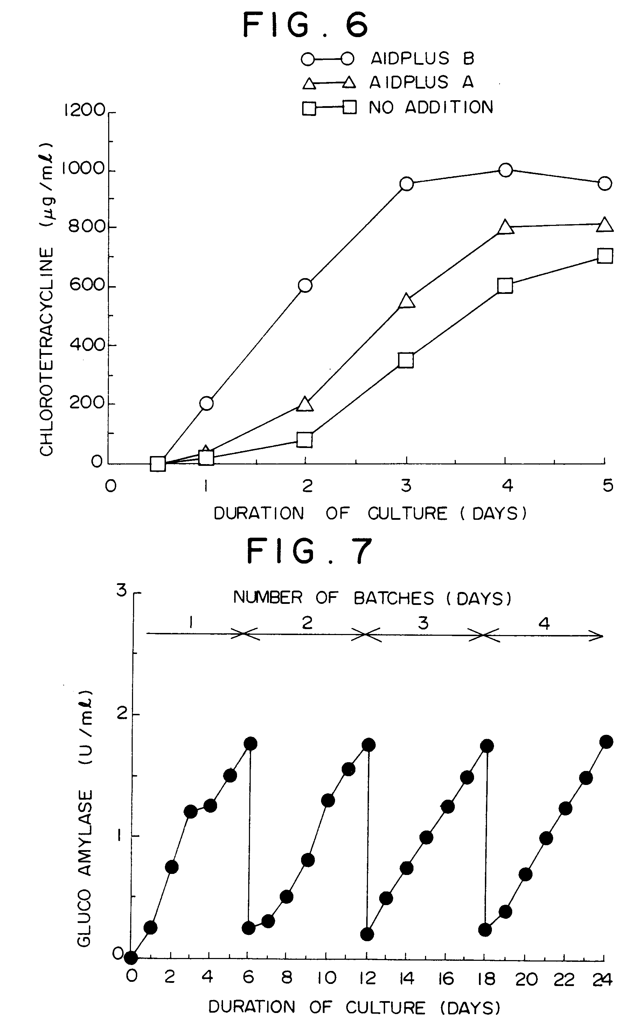

Aidplus A® and Aidplus B® that affect the fermentation of chlorotetracycline were compared with each other.
- Strain:: Sorted by single spore separation from Streptomyces aureofaciens NRRL-2209.
- Culture medium:: (Preculture) 30 g/l of sucrose, 2 g/l of ammon sulfate, 7 g/l of calcium carbonate and 16.5 ml/l of CSL were adjusted to pH 6.8, and were sterilized in an autoclave under 1 kg/cm²G for 15 minutes. (Main culture) 55 g/l of corn starch, 7 g/l of calcium carbonate, 5 g/l of ammon sulfate, 40 mg/l of heptahydrate of ferrous sulfurate, 50 ml/l of tetrahydrate of manganese sulfurate, 5 mg/l of hexahydrate of cobalt chloride, 30 mg/l of CSL, 2 g/l of soybean powder and 2.0% (v/v) of soybean oil were subjected to the pretreatment in the same manner as in Example 3, the carrier was added thereto in an amount of 1 g/l, pH was adjusted to 7.0, and the medium was sterilized under 1 kg/cm²G for 20 minutes.
- Culturing method:: A loopful of the slant of the above-mentioned strain was inoculated to a 500-ml Erlenmeyer flask and was shake-cultured in a rotary shaker (170 rpm) at 30 °C for two days to use it as a seed fungi solution. The above seed fungi solution was planted in an amount of 2% (v/v) into 500-ml Erlenmeyer flasks containing 100 ml of the main culture medium containing each of the carriers, and was shake-cultured in a rotary shaker (170 rpm) at 30 °C for five days.
- Results:: Fig. 6 shows the process of culture. Like the case of cephamycin C, addition of Aidplus B® helps increase the rate of producing the chlorotetracycline compared with the control, and the final amount of the cephamycin C production becomes greater than that of the control. In the case of Aidplus A®, the amount of production was slightly larger than that of the control. When Aidplus B® is used, it is presumed that the production of the chlorotetracycline is promoted owing to the synergistic effect of simultaneous adsorption of fungi and oils as recognized in the production by fermentation of cephamycin C.

### Example 5

### Production of gluco amylase by the packed bed reactor.

A loopful of the slant of a strain sorted by single spore separation from the gluco amylase-producing fungi, Aspergillus awamori IFO-5708, was planted in a 500-ml Erlenmeyer flask containing 100 ml of the following spore-forming culture medium that contained 1 g of Aidplus B®, and was shake-cultured in a rotary shaker (170 rpm) at 35 °C for seven days. Immobilized fungi of five flasks adsorbed by the carrier by centrifuge were all collected and were suspended in 200 ml of a production culture medium shown in Table 2, and were packed in a glass column reactor having an inner diameter of 4 cm and a height of 30 cm.

The glass column reactor was maintained at a culturing temperature of 35 °C and the aseptic air was blown at a rate of 100 ml/min through a sintered stainless steel (40 µ m ) fitted to the bottom of the reactor. After the culture was continued for six days, the ventilation was stopped so that the immobilized fungi were sedimented. Then, a portion without containing the immobilized fungi was pumped out, and a fresh culture medium was pumped into the reactor in order to carry out the culture again. Fig. 7 illustrates the results of batch culture repeated four times using the above packed bed reactor, from which it will be understood that the activity of gluco amylase was not almost lowered.

**Table 2**

| Spore formation by gluco amylase-producing fungi and enzyme-producing culture medium | | |
|---|---|---|
| | Spore-forming culture medium | Production culture medium |
| Soluble starch | 20 g/l | 20 g/l |
| CaCl₂ | 3.68 | 3.68 |
| Dextran T10 | - | 5 |
| Sodium glutamate | 0.55 | 0.55 |
| Yeast extract | 0.5 | 0.5 |
| K₂ HPO₄ | 70 mg/l | 70 mg/l |
| KH₂ PO₄ | 130 | 130 |
| MgSO₄ · 7H₂ O | 200 | 200 |
| Na₂ SO₄ · 10H₂ O | 200 | 200 |
| FeSO₄ · 7H₂ O | 20 | 20 |
| MnSO₄ · H₂ O | 10 | 10 |
| ZnSO₄ · 7H₂ O | 2 | 2 |
| CuSO₄ · 5H₂ O | 2 | 2 |
| Silicon KN75 | - | 1 ml/l |

## Claims

1. A process of culturing a filamentous fungus which comprises adding a chain clay mineral carrier to a culture medium containing the fungus or bringing the fungus immobilized on a chain clay mineral carrier into contact with a culture medium.

2. A process according to claim 1 wherein the fungus is a mold or actinomycete.

3. A process according to claim 1 or 2 wherein the chain clay mineral is a formanite-type clay.

4. A process according to claim 3 wherein the formanite-type clay is a sepiolite.

5. A process according to claim 3 wherein the formanite-type clay is in a fibrous form or a converged form.

6. A process according to any one of claims 1 to 5 which further comprises separating the fungus immobilized on the carrier from a substance produced by the fungus.

7. A process according to any one of claims 1 to 6 wherein the fungus is brought into contact with the culture medium by a batch method, a half-batch method, a repeated batch method or a continuous method.

8. A process according to any one of claims 1 to 7 wherein the culture medium is a waste material or a waste liquor containing oils and fats.

9. Use of a chain clay mineral as a carrier for immobilizing a filamentous fungus.

10. Use according to claim 9 wherein the chain clay mineral is a formanite-type clay having a fiber diameter of from 70 to 400 angstroms and a fiber length of from 0.2 to 400 µm.

11. An immobilized fungus composition which comprises a chain clay mineral carrier and a filamentous fungus immobilized thereon.

12. A composition according to claim 11 wherein the carrier has a fiber diameter of from 70 to 400 angstroms and a fiber length of from 0.2 to 400 µm, and wherein the fungus is immobilized thereon in an amount of from 0.1 to 30% by weight.
